# EUROPEAN PATENT APPLICATION

(11) **EP 2 072 012 A1**
(43) Date of publication of application: **24.06.2009**
(21) Application number: 07024500.6
(22) Date of filing: 18.12.2007
(51) Int. Cl.: A61B 6/00

(54) **Method for calibration of a camera augmented C-arm**

(71) Applicant: SIEMENS AKTIENGESELLSCHAFT, 80333 München (DE)
(72) Inventor: Heibel, Hauke, 80798 München (DE); Navab, Nassir, Professor, 81247 München (DE); Traub, Joerg, 81371 München (DE)
(74) Representative: Kley, Hansjörg

(57) **Abstract**

Method for calibration of a camera augmented C-arm (3) system comprising the steps of physically attaching multiple cameras to a gantry at predefined angles from each other, adjusting their positions such that an image captured by each camera is exactly superposed with an X-ray image, said adjusting being realized by alignment of markers (7, 8) of two sets of markers. The markers are situated in two different planes and are point symmetrical geometrical shapes. Homographies are used for certain steps of the calibration. The calibration has to be performed only once, upon system setup.

## Description

The invention relates to a method for calibration of a camera augmented C-arm, especially in a setup whereby the C-arm comprises two cameras.

Today C-arm systems, also called X-ray image intensifiers, are widely used in medical imaging, for example in orthopaedic and trauma surgery. They represent a driving technological force behind an advancement of methods for instrument guidance, used in order to reach a positioning and path as precise as possible for an instrument, especially in minimally invasive surgery. A constant aim in developments of C-arms is to reduce radiation level. Present solutions use permanent fluoroscopic imaging or C-arms with three dimensional reconstruction capabilities and external tracking systems, both resulting in a high level of radiation by needing several X-ray exposures in order to precisely determine position of the instrument. Each one of these solutions has to undergo a proprietary calibration procedure depending on special setup of components constituting the solution. Thus, the calibration is tightly bound to the setup of components and varies according to underlying method for instrument guidance used.
An enhanced system in terms of radiation dosage reduction is described in DP 10 2005 062 582.7. This system uses two cameras, superposing an image of at least one of the cameras onto an X-ray image. This system reduces the radiation dosage dramatically.
This setup has made a three dimensional positioning of the instrument possible, needing only two X-ray exposures for precise positioning of the instrument. Methods of superposing optical and X-ray images are described in US 6,227,704, US 6,229,873, US 6,447,163 and US 6,473,489.

One goal to be achieved is to be seen in calibrating the C-arm system comprising the at least two cameras, such that a desired positioning accuracy of the instrument is achieved. One way the goal is achieved is by providing a method for calibration of a camera augmented C-arm 3 system comprising the steps of,
a) physically attaching a first camera 1 to a gantry,
b) physically attaching at least one additional camera 2 arbitrarily on the C-arm 3 at a predefined angle to the first camera 1,
c) adjusting position of the first camera 1 such that the optical centre of the first camera 1 is virtually located at a constant position on a bisecting line of a beam angle of an X-ray source 6 at all times, said adjusting being realized by a first alignment of at least two first markers 7 of a first set of markers with at least two second markers 8 of a second set of markers, in both an optical image of the first camera 1 and an image of the X-ray source 6,
d) adjusting position of the second camera 2 after an orbital rotation of the C-arm 3, such that the optical centre of the additional camera 2 coincide with prior to orbital rotation optical centre of the first camera 1, said adjusting being realized by a second alignment of at least two first markers 7 of the first set of markers with at least two second markers 8 of the second set of markers, in both an optical image of the additional camera 2 after orbital rotation and the optical image of the first camera 1 prior to orbital rotation,
e) aligning an X-ray image taken after the orbital rotation of the C-arm 3 with a second image of the additional camera 2 taken prior to the orbital rotation of the C-arm 3, the aligning being realized by computing at least one homography.

Main calibration steps as described in claim 1 consist in first attaching both the first and the additional camera to the C-arm. A device comprising means for attachment of a camera and for adjustment of camera position and orientation is used therefore. For example the device comprises servo motors for moving the camera in all three space coordinates. This allows a precise calibration and presents a high potential of automatizing a production process insofar that the device is controlled by a computer.
An advantage of the presented method is that the calibration is independent of the predefined angle of the additional camera with respect to the first camera. This makes the system more flexible for various requirements, for example related to space availability within the C-arm. Step c) of claim 1 describes the adjustment of the first camera. A correct position of the first camera requires that the optical centre is virtually located at a constant position on a bisecting line of a beam angle of an X-ray source 6 at all times, in other words in the centre of the X-ray beam span. This is accomplished by double reflecting light rays using two parallel mirrors, thus yielding a parallel translation of light rays reaching the camera. One mirror is located in front of the X-ray source. This mirror is X-ray transparent. A second mirror is located in front of the first camera. If the X-ray source is near to the first camera, meaning that the second mirror intersects an X-ray beam, the second mirror has to be X-ray transparent too.
Proper adjustment of angle of the mirrors relatively to optical axis of the first camera, as well as the distance of the mirrors from each other yields a superposition of a light ray and the centre of the X-ray beam span.

One advantage of the solution as described in claim 1 is that, due to physical attachment of the first and the additional camera to the C-arm there is no need to recalibrate the system. The calibration is done once upon system production and as soon as it has been carried out, the cameras are rigidly attached to the C-arm. Furthermore, only one orbital rotation of the C-arm is required for calibration of both cameras. Within the scope of the presented method, orbital rotation means a rotation of the C-arm around a virtual centre of a virtual circle spanned by the C-arm itself for isocentric C-arms. However, the presented solution is equally applicable for non-isocentric C-arms

According to one implementation of the method according to claim 1, at least one image generated by at least one of: the first camera (1), the additional camera (2), an X-ray exposure are output on a monitoring means.
Furthermore, an X-ray image is superposed onto an optical image of one of the cameras: first camera (1), additional camera (2), thus yielding a combination of two images. By monitoring steps of calibration it is easy to take corrective actions while a calibration is still ongoing, thus accelerating a procedure of calibration. Once each of the first and additional cameras has independently been calibrated, a superposition of an X-ray image with one image of each of the said camera is done to finalize a calibration process.

According to another implementation of the method, the additional camera (2) is physically attached on the C-arm (3) orthogonally to the first camera. The advantage is that a setup using orthogonal positioning of the cameras relatively to each other yields highest precision of space information in calibration area.

In order to finalize the calibration, at least one homography step is performed. The at least one homography comprises a first sub-homography, the said first sub-homography linking the image of the X-ray source with the optical image of the first camera (2) and a second sub-homography, the said second sub-homography linking the optical image of the first camera (1) after the orbital rotation of the C-arm (3) with the optical image of the additional camera (2). A homography is defined as a mapping between a point on a plane as seen from a location outside of the said plane, to the same point on the plane as seen from a second location outside the said plane. Thus, the homography provides a method for compositing two dimensional or three dimensional objects with correct position and orientation into an image, in other words a homography is a coordinate transformation matrix. An advantage of using a homography is that the homography is a linear method, thus making computations simple.

According to yet another implementation of the method, two parallel X-ray transparent mirrors (4) are attached to the C-arm (3) such that a first mirror is located in the optical axis of the first camera (1) at a predefined angle with respect to an optical axis of the first camera and a second mirror is located in a predefined distance to the first mirror. As stated before, this setup of mirrors makes it possible to align the first camera with the X-ray source. Furthermore, a third mirror (5) is attached to the C-arm (3) such that the said third mirror is located in an optical axis of the additional camera (2) at a predefined angle with respect to the said optical axis. This advantageously makes the positioning of the additional camera very flexible with respect to available space. For example, the camera is positioned perpendicularly to the plane of the C-arm, thus allowing larger objects to be posed between the extremities of the C-arm.

Markers used for the calibration are distributed in two sets in a calibration volume, such that the first set of markers is arranged in a first plane and the second set of markers is arranged in a second plane. Each marker of the first and the second set of markers are point symmetrical geometrical shapes. This method of arrangement advantageously allows a precise calibration in a three dimensional space by aligning markers of the first plane with corresponding markers of the second plane. The first alignment is reached when a symmetry point of at least the first two markers (7) of the first set of markers coincide with a symmetry point of at least the two second markers (8) of the second set of markers in a view of the first camera. The second alignment is reached when a symmetry point of at least the two first markers (7) of the first set of markers coincide with a symmetry point of at least the two second markers (8) of the second set of markers in a view of the additional camera. The implementation of the markers as point symmetrical geometrical shapes is advantageous in that it provides an easy way for aligning markers and for monitoring a fine tuning of alignment by tracing a degree of superposition of the point of symmetry rather than superposing entire shapes. For example, the first set of markers is composed of circles and the second set of markers is composed of dots. In this case, the points of symmetry are the centre of the circles and the centres of the dots. An alignment is reached, when the dots are seen in a concentrical position with respect to the circles.

A calibration object is adapted for implementing the method according to one of the claims 1 to 12, comprising the first and the second set of markers. For example, the said calibration object comprises two light and X-ray transparent support planes on which respectively markers of the first and the second set of markers are distributed. The markers are implemented such that they are visible in a view of a camera as well as in an X-ray exposure. This advantageously renders the calibration easy to monitor, as the presence of artefacts and/or construction-related parts of the calibration object which are visible in a view of a camera and/or X-ray exposure are avoided.

The described methods are further explained by the following examples and drawings, whereby:
- Figure 1:: Side view of a two-camera augmented C-arm system
- Figure 2:: Front view of a two-camera augmented C-arm system
- Figure 3:: Rotated a two-camera augmented C-arm system during calibration phase
- Figure 4:: Side view of a calibration volume
- Figure 5:: View of a calibration volume with markers not aligned
- Figure 6:: View of a calibration object with aligned markers

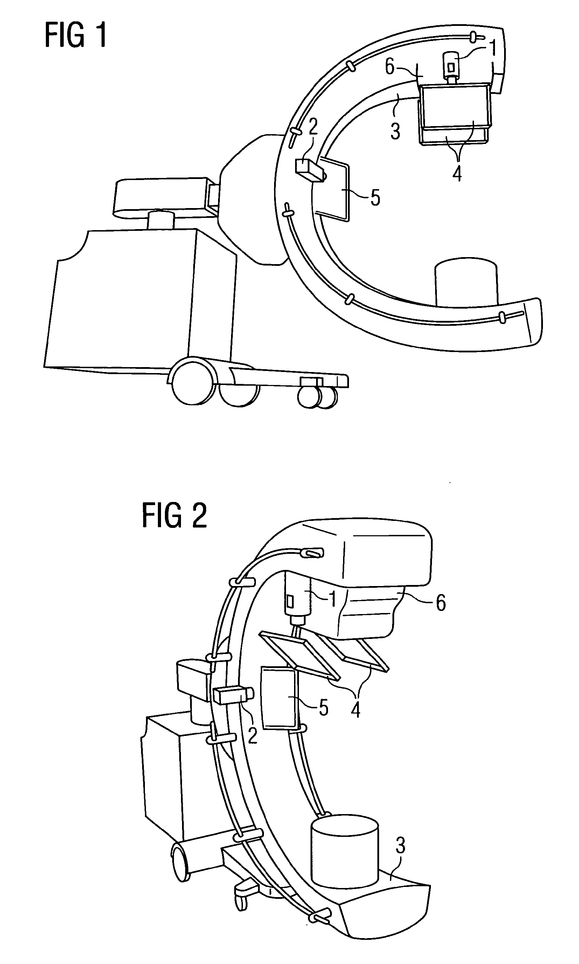

Figure 1 shows a side view of a two camera augmented mobile c-arm. A first camera (1) is attached to a gantry of an X-ray source and used for image superposition by virtually placing the first camera with two parallel X-ray transparent mirrors (4) such that it has the same optical center like the X-ray source. An additional camera (2) is attached in an orthogonal position to the first camera (1), orthogonal meaning that optical axes of the two cameras span a 90 degree angle.
Figure 2 shows a front view of the C-arm as described for figure 1.

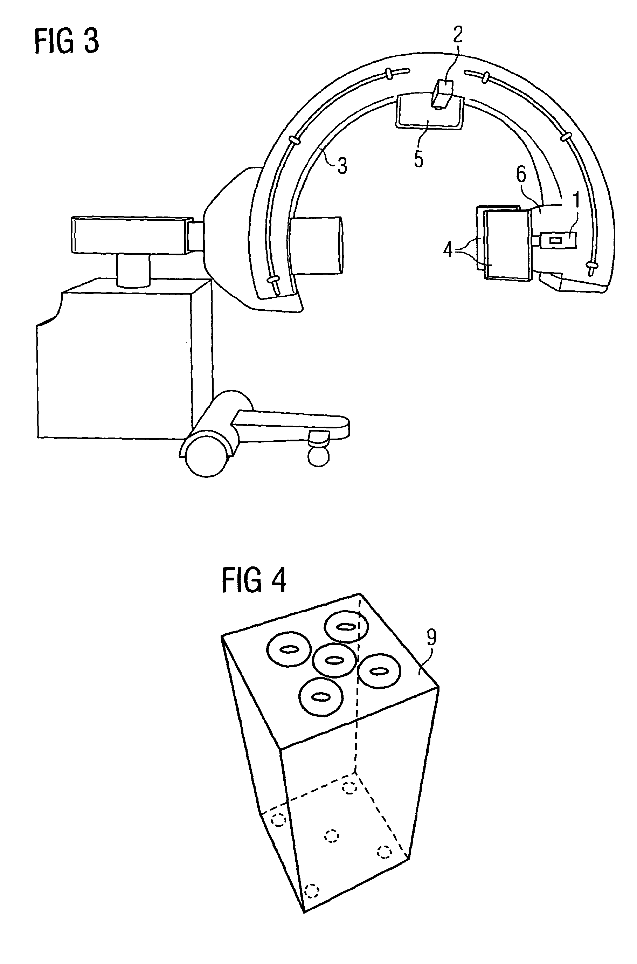

Figure 3 shows a rotated C-arm for the calibration of the additional camera (2) such that it has the same optical center as the first camera (1) before the rotation.
Figure 4 shows a calibration volume (9) comprising a first plane with a first set of markers and a second plane with a second set of markers.

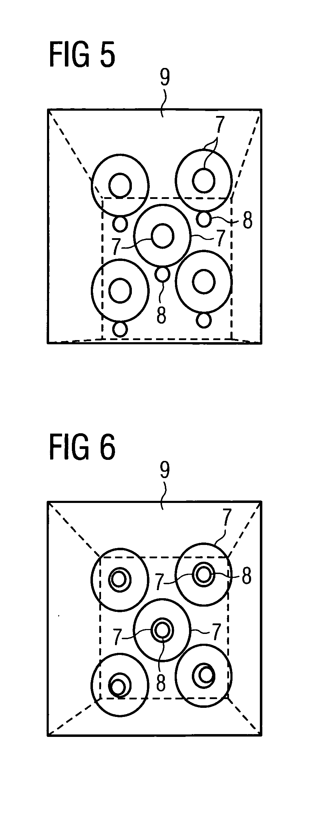

Figure 5 shows an image of the calibration volume (9) as seen from one of the first or additional cameras. The first set of markers are not aligned, as a point of symmetry of at least two first markers (7) of the first set of markers does not coincide with at least a symmetry point of corresponding two second markers (8) from the second set of markers.
Figure 6 shows an image of the calibration volume (9) as seen from one of the first or additional camera with aligned markers. In this case, the two first markers (7) of the first set of markers are aligned with the two second markers (8) of the second set of markers.

In order to emphasize that two markers of each of the two marker planes are needed for calibration, two circle pairs of each of the two marker planes in Figure 5 and Figure 6 are labelled with (7), respectively (8).

### List of Abbreviations

- 1 =: First camera
- 2 =: Additional camera
- 3 =: C-arm
- 4 =: Parallel X-ray transparent mirrors
- 5 =: Third mirror
- 6 =: X-ray source
- 7 =: Two first markers
- 8 =: Two second markers
- 9 =: Calibration volume

## Claims

1. Method for calibration of a camera augmented C-arm (3) system comprising the steps of,
a) physically attaching a first camera (1) to a gantry,
b) physically attaching at least one additional camera (2) arbitrarily on the C-arm (3) at a predefined angle to the first camera (1),
c) adjusting position of the first camera (1) such that the optical centre of the first camera (1) is virtually located at a constant position on a bisecting line of a beam angle of an X-ray source (6) at all times, said adjusting being realized by a first alignment of at least two first markers (7) of a first set of markers with at least two second markers (8) of a second set of markers, in both an optical image of the first camera (1) and an image of the X-ray source (6),
d) adjusting position of the second camera (2) after an orbital rotation of the C-arm (3), such that the optical centre of the additional camera (2) coincide with prior to orbital rotation optical centre of the first camera (1), said adjusting being realized by a second alignment of at least two first markers (7) of the first set of markers with at least two second markers (8) of the second set of markers, in both an optical image of the additional camera (2) after orbital rotation and the optical image of the first camera (1) prior to orbital rotation,
e) aligning an X-ray image taken after the orbital rotation of the C-arm (3) with a second image of the additional camera (2) taken prior to the orbital rotation of the C-arm (3), the aligning being realized by computing at least one homography.

2. Method according to claim 1, whereby at least one image generated by at least one of: the first camera (1), the additional camera (2), an X-ray exposure are output on a monitoring means.

3. Method according to claims 1 or 2, whereby an X-ray image is superposed onto an optical image of one of the cameras: first camera (1), additional camera (2), thus yielding a combination of two images.

4. Method according to one of the preceding claims, whereby the additional camera (2) is physically attached on the C-arm (3) orthogonally to the first camera.

5. Method according to one of the preceding claims, whereby the at least one homography comprises a first sub-homography, the said first sub-homography linking the image of the X-ray source with the optical image of the first camera (2).

6. Method according to one of the preceding claims, whereby the at least one homography comprises a second sub-homography, the said second sub-homography linking the optical image of the first camera (1) after the orbital rotation of the C-arm (3) with the optical image of the additional camera (2).

7. Method according to one of the preceding claims, whereby two parallel X-ray transparent mirrors (4) are attached to the C-arm (3) such that a first mirror is located in the optical axis of the first camera (1) at a predefined angle with respect to an optical axis of the first camera and a second mirror is located in a predefined distance to the first mirror.

8. Method according to one of the preceding claims, whereby a third mirror (5) is attached to the C-arm (3) such that the said third mirror is located in an optical axis of the additional camera (2) at a predefined angle with respect to the said optical axis.

9. Method according to one of the preceding claims, whereby the first and the second set of markers are distributed in a calibration volume (9) such that the first set of markers is arranged in a first plane and the second set of markers is arranged in a second plane.

10. Method according to one of the preceding claims, whereby each marker of the first and the second set of markers are point symmetrical geometrical shapes.

11. Method according to one of the preceding claims, whereby the first alignment is reached when a symmetry point of at least the two first markers (7) of the first set of markers coincide with a symmetry point of at least the two second markers (8) of the second set of markers in a view of the first camera.

12. Method according to one of the preceding claims, whereby the second alignment is reached when a symmetry point of at least the two first markers (7) of the first set of markers coincide with a symmetry point of at least the two second markers (8) of the second set of markers in a view of the additional camera.

13. Calibration object adapted for implementing the method according to one of the claims 1 to 12, comprising the first and the second set of markers.
